# EUROPÄISCHE PATENTSCHRIFT

(11) **EP 0 419 854 B1**
(45) Veröffentlichungstag und Bekanntmachung des Hinweises auf die Patenterteilung: **15.02.1995**
(21) Anmeldenummer: 90116221.4
(22) Anmeldetag: 24.08.1990
(51) Int. Cl.: A61F 9/02

(54) **Laserschutzbrille**
Laser safety glasses
Lunettes pour la protection contre laser

(30) Priorität: 26.08.1989 DE 8910235 U
(43) Veröffentlichungstag der Anmeldung: 03.04.1991
(60) Teilanmeldung: 93119161.3
(73) Patentinhaber: Firma Carl Zeiss, D-73446 Oberkochen (DE); CARL ZEISS-STIFTUNG HANDELND ALS CARL ZEISS, D-89518 Heidenheim (Brenz) (DE)
(72) Erfinder: Paysan, Heinz-Wilhelm, D-7080 Aalen-Waldhausen (DE); Grimm, Wolfgang, Dr., D-7920 Heidenheim (DE); Schürle, Hermann, D-7080 Aalen (DE); Gaiser, Hans, D-7410 Reutlingen (DE); Gutbrod, Heinz, D-7025 Leonberg 5 (DE)

(56) Entgegenhaltungen:
- DE-A- 3 111 577
- US-A- 4 703 522

## Beschreibung

Die vorliegende Erfindung betrifft eine Laserschutzbrille, die aus Schutzfiltern und einer zu deren Aufnahme dienenden, mit Ohrbügeln versehenen Brillenfassung besteht.

Solche Schutzbrillen müssen beim Arbeiten mit Lasern ab der Gefahrenklasse 3b getragen werden, um die Augen vor schädlicher Strahlung zu schützen. Dabei ist es wesentlich, daß die Schutzbrille die Augen des Benutzers nicht nur zuverlässig vor Strahlung schützt, die im wesentlichen von vorne kommt, sondern daß auch aus anderen Richtungen auftreffende Strahlung von den Augen abgehalten wird.

Dazu dienen Fassungen wie sie beispielsweise aus der DE-AS 20 62 829 bekannt sind. Diese Fassungen tragen im Schläfenbereich jeweils eine mit dem Ohrbügel verbundene Seitenblende, die im geöffneten Zustand am zugeordneten Augenrand der Fassung anliegt. Derartige Fassungen schützen nicht die Bereiche ober- und unterhalb des Augenrandes und es besteht die Gefahr, daß die Seitenblende nicht satt am zugeordneten Augenrand der Fassung anliegt und somit im kritischen Bereich ein Schlitz entsteht.

Aus dem DE-GM 85 32 493 ist eine Laserschutzbrille bekannt, bei der neben schläfenseitigen Seitenblenden auch im oberen Fassungsbereich Blenden vorgesehen sind, die den Bereich bis zur Stirn des Brillenträgers abdecken. Auch hier besteht die Gefahr, daß sich ein Schlitz zwischen Seitenblende und Augenrand der Fassung bildet; zudem sind Bereiche am unteren Rand der Fassung nicht abgeschirmt.

Diese bekannten Fassungen bauen auf den üblichen sogenannten Arbeitsschutzbrillen auf, an deren Fassung die notwendigen Blenden angesetzt werden. Dadurch werden diese Fassungen relativ teuer.

Aus der US-PS 4 527 291 ist eine mit Platten versehene Sicherheitsbrille bekannt, bei welcher die Ohrbügel von außen an der Brillenfassung befestigt sind. Diese Art der Ohrbügelbefestigung führt zu Kanten, welche ein ungewolltes Abreißen der Sicherheitsbrille ermöglichen. Außerdem besteht zwischen Brillenfassung und Brillenträgergesicht große, nicht abgedeckte Bereiche.

In letzter Zeit ist auf dem Markt die Laserschutzbrille L-04 der Firma Laser-Vision GmbH erhältlich, bei der sich die Fassung temporal bis in den Schläfenbereich erstreckt und erst dort Scharniere zum schwenkbaren Befestigen der Ohrbügel trägt. Bei dieser Fassung sind durchsichtige, mit Schutzfiltern versehene Seitenblenden und Blenden am oberen Fassungsrand vorgesehen. Da alle Blenden an einer Grundfassung gesondert befestigt sind, ist auch diese Fassung relativ teuer.

Bekannt sind auch sogenannte Korbfassungen für Laserschutzbrillen. Diese Fassungen ähneln in ihrem Aufbau den Taucherschutzbrillen, z.B. DE-PS 36 16 253. Der die Augenfassungen umgebende Fassungsteil besteht aus weichelastischem Material und deckt den Bereich zwischen den Schutzfiltern und dem Gesicht des Benutzers voll ab. Solche Korbfassungen werden meist für Besucher oder für Brillenträger verwendet, da sie über der normalen Korrekturfassung getragen werden können. Die erwähnten Korbfassungen weisen keine Ohrbügel auf; sie werden mit einem elastischen Band am Kopf des Trägers gehalten.

Die vorliegende Erfindung bezieht sich nicht auf Korbfassungen und solche sind auch ausdrücklich vom Schutz ausgenommen.

Die Aufgabe der vorliegenden Erfindung ist es vielmehr eine Laserschutzbrille, die mit Ohrbügeln ausgestattet ist so auszubilden, daß sie größtmögliche Sicherheit bei optimalem Tragekomfort bietet.

Diese Aufgabe wird erfindungsgemäß durch eine Fassung für die Laserschutzbrille gelöst, die nach den Merkmalen des Anspruchs 1 ausgebildet ist.

Diese Fassung hat den Vorteil, daß der die Schutzfilter aufnehmende Fassungsteil einstückig ausgebildet und demzufolge preiswert herstellbar ist. Dieser Fassungsteil schirmt den Bereich zwischen der Fassung und dem Gesicht des Trägers zuverlässig ab und bildet auch im Schläfenbereich Seitenblenden, d.h. er bietet Schutz vor schädlicher Strahlung die von vorn, von oben oder unten und von der Seite auftreffen kann. Dadurch, daß die Scharniere erst am ohrseitigen Ende der Seitenblenden angesetzt sind, besteht auch keine Gefahr, daß sich bei Bewegungen der Ohrbügel Schlitze im kritischen Bereich bilden.

Die Ohrbügel sind über Scharniere angesetzt, die im Inneren der Fassung angeordnet sind, wobei jeder Ohrbügel in eine Platte integriert ist, welche die Fortsetzung der zugeordneten Seitenblende des ersten Fassungsteils bildet. Dadurch ergibt sich von außen gesehen eine lange glatte Seitenblende, die nur durch eine Trennfuge unterbrochen ist. Durch die Ausbildung der Trennfuge nach Anspruch 2 ist verhindert, daß sich bei geringen Bewegungen der Ohrbügel ein für Strahlung durchlässiger Schlitz bildet.

Anspruch 3 bezieht sich auf die Ausbildung der Ohrbügel. Durch die dort beschriebene Metalleinlage ist erreicht, daß sich die Ohrbügel biegen und damit der Kopfform des Brillenträgers anpassen lassen.

Der erste, einstückig ausgebildete Fassungsteil kann aus Leichtmetall, vorzugsweise aus Aluminium hergestellt werden. Als weiteres zweckmäßiges Material hat sich der unter dem Handelsnamen "Grilamid" erhältliche Kunststoff erwiesen, der für das erste Fassungsteil glasfaserverstärkt verwendet wird. Für die Brillenbügel findet vorteilhaft auch z.B. der Kunststoff "Grilamid" Verwendung, allerdings ohne Glasfaserverstärkung und es ist gemäß Anspruch 3 eine Metalleinlage eingesetzt.

Bei Verwendung des beschriebenen Kunststoffes läßt sich bei der Laserschutzbrille nach der Erfindung eine Schutzstufe besser als 7A für die Verwendung mit UV-Lasern erreichen. Eine ähnlich hohe Schutzstufe läßt sich auch für die Verwendung von CO₂-Lasern erreichen, wenn die Fassung aus Aluminium besteht.

Es ist vorteilhaft, wenn in den Ohrbügeln gemäß Anspruch 4 Lüftungsschlitze vorhanden sind. Diese Lüftungsschlitze sind vorteilhafterweise gemäß Anspruch 5 so gestaltet, daß durch sie keine Laserstrahlung in den Bereich zwischen Laserschutzbrille und Gesicht des Brillenträgers eindringen kann. Anspruch 6 beschreibt eine zweckmäßige Ausgestaltung des Scharniers.

Das zur Schläfenplatte weisende Ohrbügelende gemäß Anspruch 7 sollte eine Rundung besitzen, deren Radiusmittelpunkt mit dem Drehpunkt des Scharniers übereinstimmt.

Desweiteren ist es vorteilhaft, die Befestigung der Ohrbügel an der Schläfenplatte der Schale mit den Schutzfiltern gemäß Anspruch 10 im oberen und unteren Bereich dieser Schläfenplatte vorzunehmen. Vorteilhafterweise besitzt die Schläfenplatte im Drehbereich der Ohrbügel gemäß Anspruch 11 eine lippenförmige Verlängerung.

Die Erfindung wird im folgenden anhand der Ausführungsbeispiele darstellenden Figuren 1 - 11 der beigefügten Zeichnungen näher erläutert. Dabei zeigen:
- Fig. 1: eine perspektivische Darstellung einer Laserschutzbrille;
- Fig. 2: die Ansicht der Laserschutzbrille nach Fig. 1 von oben;
- Fig. 3: die Ansicht der Laserschutzbrille nach Fig. 1 von unten;
- Fig. 4: einen Schnitt entlang der Linie IV-IV der Fig. 1;
- Fig. 5: eine Teilansicht der Laserschutzbrille von oben mit teilweise eingeklapptem Ohrbügel;
- Fig. 6: eine perspektivische Darstellung einer Laserschutzbrille mit Lüftungsschlitzen;
- Fig. 7: einen Ohrbügel mit Lüftungsschlitzen im Schnitt;
- Fig. 8: einen zweiten Ohrbügel mit Lüftungsschlitzen im Schnitt;
- Fig. 9: einen Ohrbügel in seitlicher Ansicht;
- Fig. 10: einen Scharnierbereich im Schnitt von oben gesehen; und
- Fig. 11: einen Scharnierbereich im Schnitt von oben gesehen.

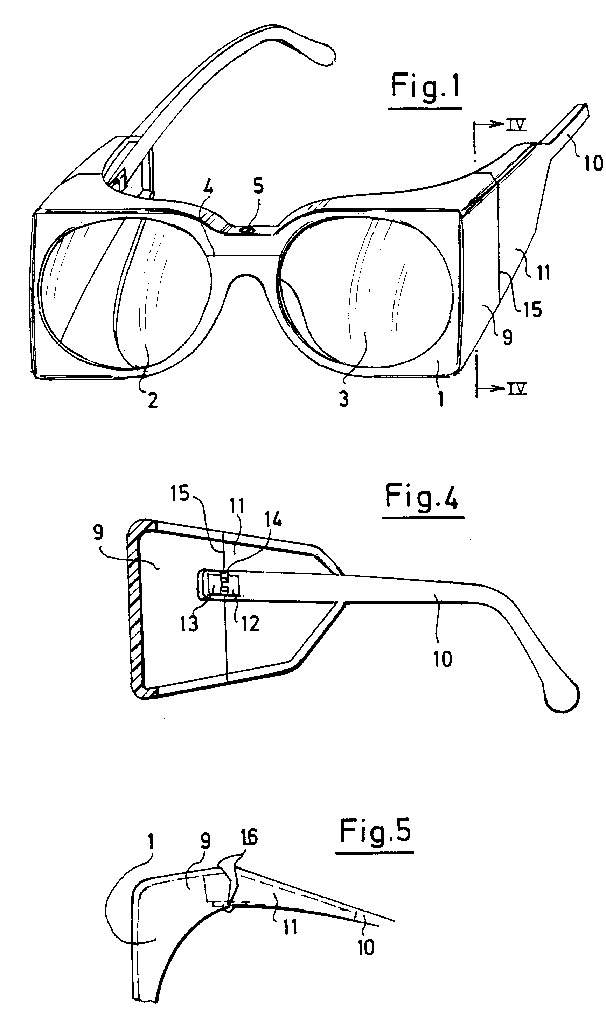

In Figur 1 ist das erste Fassungsteil der Laserschutzbrille mit (1) bezeichnet. Dieses Fassungsteil ist einstückig ausgebildet und trägt die Schutzfilter (2 und 3). Zum Einsetzen der Schutzfilter (2, 3) kann das Fassungsteil (1) bei (4) geschlitzt sein. Eine Schraube (5) dient nach dem Einsetzen der Schutzfilter (2, 3) zum strahlungsdichten Schließen des Schlitzes (4).

Wie insbesondere aus den Fig. 1, 2 und 3 ersichtlich ist ist das Fassungsteil (1) beidseitig temporal bis in den Schläfenbereich des Brillenträgers hineingezogen und bildet dort Seitenblenden, die auch als Schläfenplatten bezeichnet werden können.

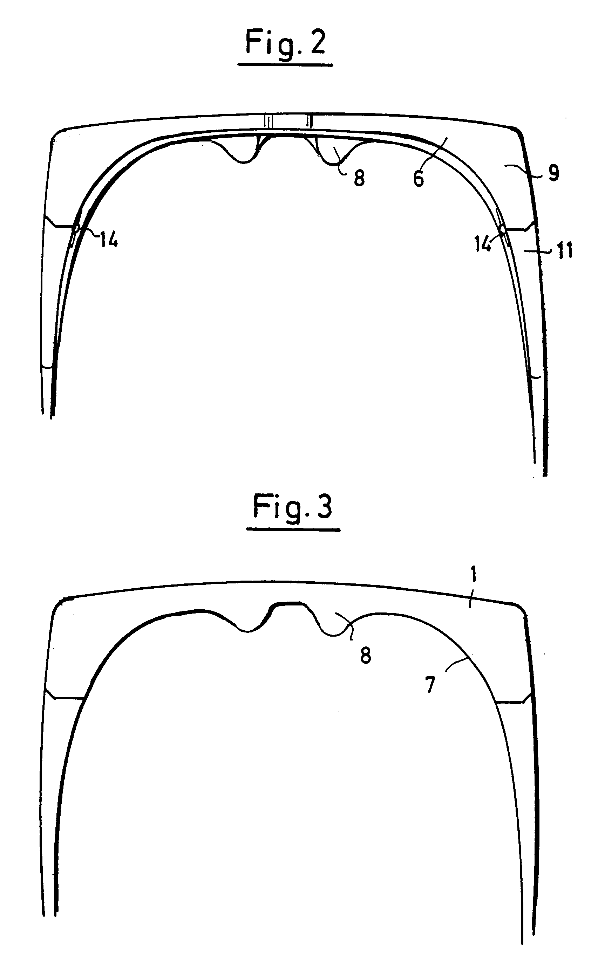

Wie Fig. 2 zeigt ist mit dem Fassungsteil (1) eine obere Schutzblende (6) integriert, welche den Bereich zwischen der Fassung und der Stirn des Brillenträgers abdeckt.

Aus Fig. 3 ist ersichtlich, daß der untere Teil des Fassungsteils (1) als Schutzblende (7) ausgebildet ist, welche den Bereich zwischen der Fassung und dem Gesicht des Brillenträgers überdeckt. Dabei kann es vorteilhaft sein, diese Schutzblende (7) so auszubilden, daß aus Gründen der Belüftung ein kleiner Schlitz zwischen dem Gesicht des Brillenträgers und der Blende (7) verbleibt. Die untere Blende (7) geht direkt in die Nasenauflagen (8) über. Auch diese sind in das Fassungsteil (1) integriert.

Wie aus diesen Figuren ersichtlich ist, bildet das Fassungsteil (1) im Querschnitt eine zum Gesicht des Brillenträgers hin offene Schale, in deren Frontfläche die Schutzfilter eingesetzt sind.

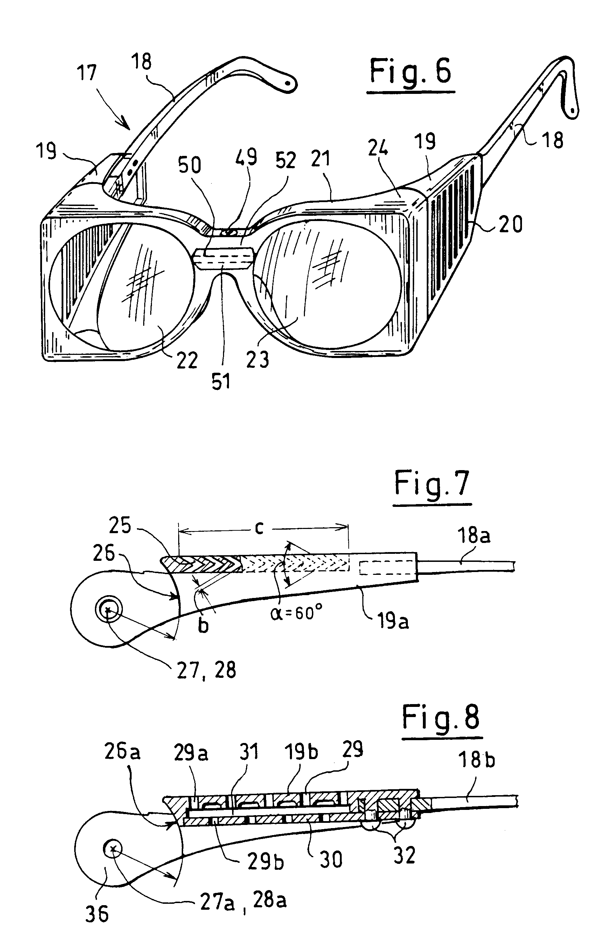

An jeder Schläfenplatte (9) ist ein Ohrbügel (10) drehbar befestigt. Wie Fig. 4 zeigt ist der Ohrbügel (10) in eine Platte (11) integriert, welche die Fortsetzung der Schläfenplatte (9) bildet. Mit der Schläfenplatte (9) ist ein erster Scharnierteil (12) fest verbunden, während der zweite Scharnierteil (13) mit der Platte (11) fest verbunden ist. Der Scharnierbolzen (14) bildet den Drehpunkt des Scharniers. Er ist auf der Innenseite der Fassung (1) angeordnet und zwar in der Ebene der Trennfuge (15) zwischen den beiden Platten (9 und 11).

Wie Fig. 5 zeigt ist die Trennfuge so ausgebildet, daß sie abgeschrägte Teile (16) enthält, welche jeweils über die gesamte Wandstärke der beiden Platten (9 und 11) reichen. Diese beiden Abschrägungen (16) liegen bei geöffnetem Ohrbügel aufeinander. Durch diese Ausbildung der Trennfuge ist erreicht, daß auch bei nicht ganz geöffnetem Ohrbügel kein Schlitz entsteht, welcher für Strahlung durchlässig ist.

Die in Figur 6 dargestellte Laserschutzbrille (17) besitzt an ihren Ohrbügeln (18) jeweils eine Platte (19) mit Lüftungsschlitzen (20). Diese Lüftungsschlitze (20) ermöglichen eine gute Belüftung im Bereich zwischen Laserschutzbrille (17) und dem Gesicht des Brillenträgers und verhindern einen Hitzestau unter der Laserschutzbrille (17). Die Schutzfilter (22, 23) sind objektseitig konvex gewölbt, um durch divergente Reflexion die reflektierte Strahlungsdichte zu verringern. Das Fassungsteil (21) im Bereich der Schutzfilter (22, 23) weist deshalb zweckmäßigerweise auch eine entsprechende Wölbung auf. Im Fassungsteil (21) befindet sich zwischen den beiden Schutzfiltern (22, 23) ein waagrechter Schlitz (50), der zur Montage und Demontage der Schutzfilter (22, 23) notwendig ist. Nachdem die Montage der Schutzfilter (22, 23) in das Fassungsteil (21) der Laserschutzbrille (17) erfolgt ist, wird in den Schlitz (50) ein Abdeckungsteil (51) eingeführt. Dieses Abdeckungsteil (51) ist frontseitig verbreitert ausgeführt. Eine Schraube (49) geht vertikal durch den Nasensteg (52) und durch den Schlitz (50), wobei sie das eingesteckte Abdeckungsteil (51) fest im Schlitz (50) verankert. Die genaue Gestaltung des Ohrbügels (18) ist in den folgenden Fig. 7-9 dargestellt.

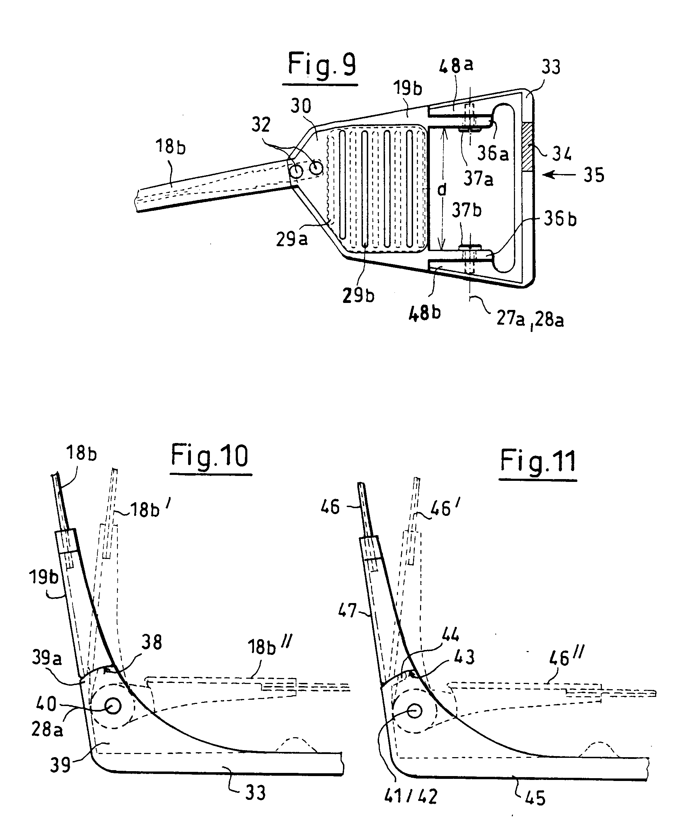

Das Fassungsteil (21) der Laserschutzbrille (17) mit den Schutzfiltern (22, 23) ist, bis auf den Bereich an den Ohrbügeln (17), wie in den Fig. 1-5 beschrieben gestaltet. Die unterschiedliche Befestigung der Ohrbügel (17) an der Schläfenplatte (24) des Fassungsteil (21) wird anhand der Fig. 10 und 11 näher erläutert.

In Fig. 7 ist ein Ohrbügel (18a) dargestellt, welcher in der an ihm angebrachten Platte (19a) seitlich angebrachte Lüftungsschlitze (25) besitzt. Diese Lüftungsschlitze (25) haben eine Breite (b) und sind V-förmig ausgebildet mit einem Öffnungswinkel α = 60°. Durch die V-förmige Gestalt der Lüftungsschlitze (25) wird sichergestellt, daß keine Lichtstrahlung durch die Lüftungsschlitze (25) dringen kann. Die Lüftungsschlitze (25) stellen somit eine Lichtfalle dar. Sie sind dabei auf einen Bereich (c) der Platte (19a) begrenzt. Die Platte (19a) am Ohrbügel (18a) endet scharnierseitig mit einer gerundeten Fläche (26), deren Radiusmittelpunkt (27) mit dem Drehmittelpunkt (28) des Scharniers zusammenfällt.

Auch bei dem in Fig. 8 dargestellten Ohrbügel (18b) endet die am Ohrbügel (18a) angebrachte Platte (19a) scharnierseitig mit einer gerundeten Fläche (26), deren Radiusmittelpunkt (27a) mit dem Drehmittelpunkt (28a) des Scharniers zusammenfällt. Die am Ohrbügel (18a) angebrachte Platte (19a) besitzt sowohl äußere (29a) als auch innere (29b) Lüftungsschlitze (29). Die äußeren und inneren Lüftungsschlitze (29) befinden sich sowohl an der am Ohrbügel (18a) angebrachten Platte (19a) als auch an einer diskreten zweiten Platte (30) und sind durch einen Lüftungskanal (31) miteinander verbunden. Dabei sind die äußeren und inneren Lüftungsschlitze (29a, b) so zueinander angeordnet, daß ein Lichtstrahl nicht direkt von außen nach innen durch die Lüftungsschlitze (29) fallen kann. Die am Ohrbügel (18b) befestigte Platte (19b) besitzt am hinteren Ende zwei als Nieten (32) nutzbare Körper. Mit diesen Nieten (32) wird sowohl die zweite diskrete Platte (30) als auch der Ohrbügel (18b) an der Platte (19b) befestigt.

In Fig. 9 ist ein Ohrbügel (18b) mit an ihm angebrachter Platte (19b) und Lüftungsschlitzen (29a, b) gemäß Fig. 8 in seitlicher Ansicht von innen mit durchgetrenntem Nasensteg (34) am Fassungsteil (33) der Laserschutzbrille (35) dargestellt. Deutlich ist hier zu sehen, daß die äußeren und inneren Lüftungsschlitze (29a, b) zueinander versetzt angeordnet sind.

Die Platte (19b) besitzt zwei Scharnierteile (36 bzw. 36a, 36b). Mit diesen Scharnierteilen (36) wird der Ohrbügel (18b) im oberen und unteren Bereich der Schläfenplatte (48a, b) drehbar befestigt. Dies erfolgt durch zwei Schrauben (37a, 37b), deren Gewinde nur in jeweils eine Gewindebohrung in der Schläfenplatte (48a, b) greift. Zwischen den beiden Scharnierteilen (36) besteht ein Abstand (d).

In Figur 10 ist der Ohrbügel (18b) mit an ihm angebrachter Platte (19b) aus Figur 8 und 9 in Ansicht von oben dargestellt. Die kreisrunde Gleitfläche (38) zwischen der Platte (19b) am Ohrbügel (18b) und der Schläfenplatte (39) des Fassungsteils (33) hat ihren Radiusmittelpunkt (40) im Drehmittelpunkt (28a) des Drehscharniers. Da die Schläfenplatte (39) in der Nähe der Gleitfläche (38) eine Verdickung (39a) besitzt, kann der Ohrbügel (18b) bis zur Stellung (18b') bewegt werden, ohne daß Strahlung im Bereich der Gleitfläche (38) eindringen kann. In der Stellung (18b'') befindet sich die Laserschutzbrille in ihrer Aufbewahrungsstellung.

Sollte es notwendig sein, daß man den Bügel (18b) über die in Fig. 10 dargestellte Stellung (18b') bewegen kann, ohne daß Lichtstrahlung an der Gleitfläche (38) ins Innere der Brillenfassung eindringen kann, so kann man die Verdickung (39a) auch noch vergrößern. Dies ist in Fig. 11 dargestellt.

In Fig. 11 kann man den Brillenbügel (46) noch über die Stellung (46') hinausbewegen, ohne daß Lichtstrahlung in das Innere der Laserschutzbrille (45) eindringen kann. Die Laserschutzbrille (45) besitzt an der Gleitfläche (43), deren Radiusmittelpunkt (41) auch hier im Drehmittelpunkt (42) des Drehscharniers ist, eine lippenartige Verlängerung (44). An dieser lippenartigen Verlängerung (44) kann die Platte (47) des Ohrbügels (46) entlanggleiten. Die Verlängerung (44) befindet sich dabei im Bereich (d) zwischen den beiden Scharnierteilen (36a, b in Fig. 9) an der Platte (47). In ihrer Aufbewahrungsstellung (46'') kann die Laserschutzbrille (45) in ihr Etui (hier nicht dargestellt) gesteckt werden.

Die in den Figuren dargestellte neue Laserschutzbrille kann beispielsweise aus Aluminium hergestellt werden. Es ist auch möglich die Fassung aus einem Kunststoff, vorzugsweise aus dem unter der Handelsbezeichnung "Grilamid" erhältlichen Kunststoff auszubilden. Dabei wird zweckmäßig das Fassungsteil aus glasfaserverstärktem Kunststoff gebildet, während die Ohrbügel und die integrierte Platte aus nichtglasfaserverstärktem Kunststoff gebildet werden. Weiterhin ist es vorteilhaft, die Ohrbügel in hier nicht dargestellter Weise mit einer Metalleinlage zu versehen, um sie in gewissem Umfang verformbar zu halten, so daß die Fassung dem Kopf des Brillenträgers genau angepaßt werden kann. Die in den Fig. 7 und 8 dargestellte Formgestaltung der Lüftungsschlitze ist nur beispielhaft. Alle Formgestaltungen von Lüftungsschlitzen, welche einen direkten Durchgang von Lichtstrahlung verhindern, können verwendet werden.

## Patentansprüche

1. Laserschutzbrille, bestehend aus einer mit Ohrbügeln versehenen Brillenfassung, in welcher Schutzfilter (2, 3) eingesetzt sind, wobei die Fassung (11) einstückig ausgebildet ist und im Querschnitt die Form einer zum Gesicht des Brillenträgers offene Schale hat, in deren Frontfläche die Schutzfilter (2, 3) eingesetzt sind und deren obere (6) und unteren Flächen (7) den Bereich bis zum Gesicht des Brillenträgers überdecken, wobei die Schale sich beidseitig temporal bis in den Schläfenbereich des Brillenträgers erstreckt und dort jeweils eine Schläfenplatte (9) bildet, auf deren Innenseite ein Scharnier (12, 13, 14) zur drehbaren Befestigung eines Ohrbügels (10) befestigt ist und wobei jeder Ohrbügel (10) an seinem dem Scharnier zugewandten Ende in die Innenseite einer Platte (11) integriert ist, welche im Zustand des geöffneten Ohrbügels (10) eine Fortsetzung der Schläfenplatte (9) bildet, so daß sich aus Schläfenplatte (9) und Platte (11) eine lange glatte Seitenblende ergibt.

2. Laserschutzbrille nach Anspruch 1, dadurch gekennzeichnet, daß im Bereich der Trennfuge (15) zwischen Schläfenplatte (9) und der mit dem Ohrbügel (10) verbundenen Platte (11) das Material dieser Platten über die gesamte Wandstärke so abgeschrägt ist, daß die Abschrägungen (16) bei geöffnetem Ohrbügel (10) aufeinanderliegen.

3. Laserschutzbrille nach einem der Ansprüche 1 und 2, dadurch gekennzeichnet, daß jeder Ohrbügel (10) mit einer Metalleinlage versehen ist.

4. Laserschutzbrille nach einem der Ansprüche 1-3, dadurch gekennzeichnet, daß in der mit dem Ohrbügel (18, 18a, 18b) verbundenen Platte ((19, 19a, 19b) Lüftungsschlitze (20, 25, 29) angebracht sind.

5. Laserschutzbrille nach Anspruch 4, dadurch gekennzeichnet, daß die Lüftungsschlitze (20, 25, 29) lichtstrahlungsundurchlässig ausgebildet sind.

6. Laserschutzbrille nach einem der Ansprüche 1-5, dadurch gekennzeichnet, daß der Drehpunkt (14) des Scharniers (12, 13, 14) auf der Innenseite der Trennfuge (15) liegt.

7. Laserschutzbrille nach einem der Ansprüche 1-5, dadurch gekennzeichnet, daß zwischen jeweils einer Platte (19, 19a, 19b) an jeweils einem Ohrbügel (18, 18a, 18b) und dem Fassungsteil (21, 33) der Laserschutzbrille (17, 45) eine kreisrunde Gleitfläche (26, 26a, 38, 44) angebracht ist, deren Radiusmittelpunkt (27, 27a, 40, 41) mit dem Drehmittelpunkt (28, 28a, 42) des Scharniers zusammenfällt.

8. Laserschutzbrille nach Anspruch 7, dadurch gekennzeichnet, daß der Drehpunkt (28, 28a, 42) des Scharniers sich im Bereich der Schläfenplatte (24, 48a, b; 39) befindet.

9. Laserschutzbrille nach einem der Ansprüche 1-8, dadurch gekennzeichnet, daß zur Befestigung einer Platte (19; 19a, b) an einem Ohrbügel (18, 18a, 18b) zwei Scharnierteile (36a, b) an der Platte (19; 19a, b) ausgebildet sind.

10. Laserschutzbrille nach Anspruch 9, dadurch gekennzeichnet, daß die Scharniere zur Befestigung der Platte (19; 19a, b) im oberen und unteren Bereich der Schläfenplatte (24; 48a, b; 39) angeordnet sind.

11. Laserschutzbrille nach einem der Ansprüche 8 oder 9, dadurch gekennzeichnet, daß im Inneren des Fassungsteils der Laserschutzbrille (45) im Bereich der Gleitfläche (43) eine lippenartige Verlängerung (44) im nicht von den Scharnieren abgedeckten Raum ausgebildet ist.

## Claims

1. Safety spectacles against laser radiation consisting of protective filters (2, 3) and a spectacle frame (11) serving to accommodate said filters (2, 3) and being provided with side arms, wherein the frame (11) is of integral construction and has in cross section the form of a dish which is open towards the spectacle-wearer's face, in the front surface of the frame the protective filters (2, 3) are inserted and the top (6) and bottom surfaces (7) of the frame cover the area as far as the spectaclewearer's face, wherein the dish extends temporally on both sides into the spectacle-wearer's temple area and forms a temple plate (9) there in each case, on the inside of each temple plate a hinge (12, 13, 14) is attached for pivotable attachment of a side arm (10) , and wherein each side arm (10) is integrated at its end facing the hinge in the inside of a plate (11) which forms a continuation of the temple plate (9) in the state of the opened side arm (10), so that the temple plate (9) and the plate (11) form a long smooth side shield.

2. Safety spectacles against laser radiation as claimed in claim 1, wherein, in the area of the separation joint (15) between temple plate (9) and the plate (11) connected to the side arm (10), the material of these plates is sloped over the entire wall thickness in such a way that the sloping surfaces (16) rest on each other when the side arm (10) is opened.

3. Safety spectacles against laser radiation as claimed in one of claim 1 and 2, wherein each side arm (10) is provided with a metal insert.

4. Safety spectacles against laser radiation as claimed in one of claims 1-3, wherein ventilation slits (20, 25, 29) are made in the plate (19, 19a, 19b) connected to the side arm (18, 18a, 18b).

5. Safety spectacles against laser radiation as claimed in claim 4, wherein the ventilation slits (20, 25, 29) are designed to be impermeable to light radiation.

6. Safety spectacle against laser radiation as claimed in one of claims 1-5, wherein the pivot point (14) of the hinge (12, 13, 14) lies in the inside of the separation joint (15).

7. Safety spectacles against laser radiation as claimed in one of claim 1-5, wherein a circular sliding surface (26, 26a, 38, 44), the center of radius (27, 27a, 40, 41) of which coincides with the center of pivoting (28, 28a, 42) of the hinge, is attached between a plate (19, 19a, 19b) in each case, respectively on a side arm (18, 18a, 18b) and the frame part (21, 33) of the safety spectacles (17, 45) against laser radiation.

8. Safety spectacles against laser radiation as claimed in claim 7, wherein the pivot point (28, 28a, 42) of the hinge is located in the area of the temple plate (24, 48a, b; 39).

9. Safety spectacles against laser radiation as claimed in one of claims 1-8, wherein two hinge parts (36a, b) are formed on the plate (19; 19a, b) to a side arm (18, 18a, 18b).

10. Safety spectacles against laser radiation as claimed in claim 9, wherein the hinges for attaching the plate (19; 19a, b) are arranged in the upper and lower area of the temple plate (24, 48a, b; 39).

11. Safety spectacles against laser radiation as claimed in one of claims 8 or 9, wherein a lip-like extension (44) is formed in the interior of the frame part of the safety spectacles (45) against laser radiation in the region of the sliding surface (43), in the space not covered by the hinges.

## Revendications

1. Lunettes de protection contre le laser, constituée par une monture pourvue de branches et dans laquelle sont insérés des filtres protecteurs (2,3), et dans lesquels la monture (11) est réalisée d'un seul tenant et possède, en coupe transversale, la forme d'une coque ouverte en direction de la personne portant les lunettes et dans la surface avant de laquelle sont insérés les filtres protecteurs (2,3), et dont la surface supérieure (6) et la surface inférieure (7) recouvrent la zone s'étendant jusqu'au visage de la personne portant les lunettes, et dans lesquelles la coque s étend des deux côtés jusque dans la zone des tempes de la personne portant des lunettes et forme, en cet endroit, respectivement une plaque temporale (9), sur la face intérieure de laquelle est fixée une charnière (12,13,14) permettant la fixation, avec possibilité de pivotement, d'une branche de lunette (10), et chaque branche de lunette (10) est intégrée, au niveau de son extrémité tournée à l'opposé de la charnière, dans la face intérieure d'une plaque (11), qui, lorsque la branche de lunette (10) est ouverte, forme un prolongement de la plaque temporale (9) de sorte que la plaque temporale (9) et la plaque (11) forment un long panneau latéral lisse.

2. Lunettes de protection contre la laser selon la revendication 1, caractérisées en ce que dans la zone du joint de séparation (15) entre la plaque temporale (9) et la plaque (11) reliée à la branche de lunette (10), le matériau de ces plaques est biseauté sur la totalité de l'épaisseur de paroi de sorte que les biseaux (16) s'appliquent l'un contre l'autre lorsque la branche de lunette (10) est ouverte.

3. Lunettes de protection contre la laser selon l'une des revendications 1 et 2, caractérisées en ce que chaque branche de lunette (10) comporte un insert métallique.

4. Lunettes de protection contre la laser selon l'une des revendications 1-3, caractérisées en ce que des fentes d'aération (20,25,29) sont aménagées dans la plaque (19,19a,19b) reliée à la branche de lunette (18,18a,18b).

5. Lunettes de protection contre la laser selon la revendication 4, caractérisées en ce que les fentes d'aération (20,25,29) sont réalisées de manière à être opaques à la lumière.

6. Lunettes de protection contre la laser selon l'une des revendications 1-5, caractérisées en ce que le centre de rotation (14) de la charnière (12,13,14) est situé sur la face intérieure du joint de séparation (15).

7. Lunettes de protection contre la laser selon l'une des revendications 1-5, caractérisées en ce qu'entre respectivement une plaque (19,19a,19b) située respectivement sur une branche de lunette (18,18a,18b) et la partie formant monture (21,33) des lunettes de protection contre le laser (17,45) est disposée une surface circulaire de glissement (26,26a,38,44), dont le centre (27,27a,40,41) du rayon coïncide avec le centre de rotation (28,28a,42) de la charnière.

8. Lunettes de protection contre la laser selon la revendication 7, caractérisées en ce que le centre de rotation (28,28a,42) de la charnière est situé dans la zone de la plaque temporale (24,48a,b; 39).

9. Lunettes de protection contre la laser selon l'une des revendications 1-8, caractérisées en ce que pour la fixation d'une plaque (19;19a,19b) sur une branche de lunette (18,18a,18b), deux éléments de charnière (36a,b) sont formés sur la plaque (19;19a,b).

10. Lunettes de protection contre la laser selon la revendication 9, caractérisées en ce que les charnières servant à fixer la plaque (19,19a,b) sont disposées dans la partie supérieure et dans la partie inférieure de la plaque temporale (24;48a,b;39).

11. Lunettes de protection contre la laser selon l'une des revendications 8 ou 9, caractérisées en ce qu'un prolongement en forme de lèvre (44) est formé, dans l'espace non recouvert par les charnières, à l'intérieur de la partie formant monture des lunettes de protection contre le laser (45), au niveau de la surface de glissement (43).
